# EUROPEAN PATENT SPECIFICATION

(11) **EP 1 567 091 B1**
(45) Date of publication and mention of the grant of the patent: **28.09.2011**
(21) Application number: 03783584.0
(22) Date of filing: 17.11.2003
(51) Int. Cl.: A61F 2/06

(54) **STENT-GRAFT ATTACHMENT**
BEFESTIGUNG FÜR EINEN STENT-GRAFT
FIXATION D'ENDOPROTHESES

(30) Priority: 04.12.2002 US 309731
(43) Date of publication of application: 31.08.2005
(73) Proprietor: Boston Scientific Limited, St. Michael (BB)
(72) Inventor: MCCULLAGH, Orla, Waterloo, MA 02472 (US); THISTLE, Robert, Brockton, MA 02302 (US)
(74) Representative: Hauck Patent- und Rechtsanwälte
(86) International application number: PCT/US2003/036675
(87) International publication number: WO 2004/049975

(56) References cited:
- EP-A- 0 824 899
- WO-A-97/25002
- FR-A- 2 833 153
- US-A- 5 609 627
- US-B1- 6 280 466

## Description

### FIELD OF THE INVENTION

This invention relates generally to stent-grafts, and more particularly to an apparatus and method for attaching a covering or graft to intersecting stent members.

### BACKGROUND OF THE INVENTION

Stent-grafts are used in surgical procedures to treat various vascular defects.

Stent-grafts comprise a combination of a graft or covering with a stent or stent frame providing structural support to the graft or covering. The stent in such combinations is generally tubular and typically comprises an open framework or mesh of structural elements such as wires or thin metallic members, which may cross or intersect one another in various ways. In one stent graft configuration, a braided stent is provided where opposing helical stent members overlap one another to form crossing intersections. The braided stent is designed to contract radially for endoluminal placement into a patient and expand radially into a configuration in which it comprises an open lumen. The graft associated with a stent-graft is a covering or liner, disposed inside or outside of the stent and covering the stent framework to define a fluid passageway through the lumen of the stent.

In another stent configuration, US patent No. 5,609,627 to Goicoechca et al. discloses a bifurcated endoluminal prosthesis that includes a plurality of hoop members coupled to adjacent hoop members at their respective apices. A graft layer covers the hoop members. The graft layer may be attached to the stent by stitching Filaments around the apices.

US 6,280,426 B1 discloses a graft system comprising several stent sections made of zigzag shape struts. The stent sections are spaced apart and are affixed to a covering material via blanket stitches.

WO 97/25002 A1 discloses an expansible endoluminal prothesis compring a tubular frame including a plurality of ring frames, the tubular frame supporting an inner liner sutured to the frame.

### SUMMARY OF THE INVENTION

The present invention is directed to a stent-graft according to claim 1 and a method of attaching a covering to a stent according to claim 14.

In accordance with an exemplary embodiment of the present invention, an attachment for securing a stent covering or graft to intersecting stent members includes a continuous filament repeatedly passing through the stent covering and wrapping around a stent member adjacent an intersection of that member with another member. The continuous filament is knotted around the intersection thereof, and then continues repeatedly passing through the covering and wrapping around the intersecting stent member. In an exemplary embodiment, the filament is a suture, the intersecting stent members form the end of a braided stent, and the covering comprises laminated layers of a woven dacron and a thin expanded polytetrafloroethlene.

The present invention may provide attachment of a vascular graft to a variety of stent configurations, while allowing the stent members to hinge or pivot freely without forming wrinkles and crimps in the graft. According to an exemplary embodiment, the continuous filament is knotted at a plurality of adjacent intersections circumscribing a stent end formed by the intersecting stent members and lashed to a circumferential succession of intersecting stent members. This lashing between the stent members entails the filament repeatedly passing through the covering, and looping around portions of the stent members interconnecting the adjacent intersections. The ends of the covering may follow the contour of the frame members between the selected crossing interactions. Each of the lashing loops may comprise one of several sequential loops on the respective intersecting stent members, with interposed loops of the filament, or optionally some other restraining member, such as a ring or wire loop, surrounding the interposed intersections, preferably longitudinally.

An attachment may also be provided at an end of a braided stent section comprising alternating crossing and crimped intersections. The lashing is again performed between alternating crossing and crimped intersections. The filament is wrapped around a portion of the stent members and then knotted longitudinally around the intersecting stent members at crossing intersections and horizontally around the intersecting stent members at crimped intersections.

An attachment may also be provided at an end of a braided section of a stent formed by reversing the axial direction of the stent members. The continuous filament is knotted at a plurality of adjacent intersections circumscribing a stent formed by the stent members. The filament repeatedly passes through the covering and loops around the reversing portions of the stent members (i.e., turns), interconnecting the adjacent intersections. The contour of the frame members may be followed with the ends of the covering between selected crossing intersections.

### BRIEF DESCRIPTION OF THE DRAWINGS

FIG. 1 is an iliac extension of a bifurcate stent-graft;
FIG. 2 is a bifurcate section of a bifurcate stent-graft;
FIG. 3 shows the proximal end of the iliac extension of Fig. 1 prior to attaching the covering to the intersecting stent members;
FIG. 4 shows the proximal end of the iliac extension of Fig. 1 with the covering attached to the intersecting stent members with a continuous filament in accordance with an exemplary embodiment of the present invention;
FIG. 5a shows a first step of a method for attaching the covering to the intersecting stent members at the proximal end of the iliac extension of Fig. 1 according to an exemplary embodiment of the present invention;
FIG. 5b shows a second step of a method for attaching the covering to the intersecting stent members at the proximal end of the iliac extension of Fig. 1 according to an exemplary embodiment of the present invention;
FIG. 5c shows a third step of a method for attaching the covering to the intersecting stent members at the proximal end of the iliac extension of Fig. 1 according to an exemplary embodiment of the present invention;
FIG. 5d shows a fourth step of a method for attaching the covering to the intersecting stent members at the proximal end of the iliac extension of Fig. 1 according to an exemplary embodiment of the present invention;
FIG. 5e shows a fifth step of a method for attaching the covering to the intersecting stent members at the proximal end of the iliac extension of Fig. 1 according to an exemplary embodiment of the present invention;
FIG. 6 shows, from inside the stent, an attachment at one intersection featuring a knot formed by the continuous filament fixing the covering to the intersecting stent members according to an exemplary embodiment of the present invention;
FIG. 7 shows, from outside the stent, an attachment at one intersection featuring the continuous filament with a knot fixing the covering to the intersecting stent members;
FIG. 8 shows the distal end of the iliac extension of Fig. 1 with crimped intersections having crimps holding together the intersecting stent members;
FIG. 9 shows a continuous lashing, a longitudinal knot at selected crossing intersections, and a lateral knot directly below selected crimped intersections of the stent members;
FIG. 10 shows a step in a method for forming an attachment at the proximal end of the bifurcate stent-graft according to an exemplary embodiment of the present invention;
FIG. 11 shows a step, subsequent to the step illustrated in FIG. 10, in a method for forming an attachment at the proximal end of the bifurcate stent-graft according to an exemplary embodiment of the present invention;
FIG. 12 shows the step illustrated in FIG. 11 from outside the stent;
FIG. 13 shows a step in a method for forming an attachment conforming to axially reversing segments of intersecting stent members according to an exemplary embodiment of the present invention;
FIG. 14 shows a completed attachment of FIG. 13; and
FIG. 15 shows a covering attached to intersecting stent members with a continuous filament and a pivotal restraint in accordance with an alternate exemplary embodiment of the present invention.

While the applicant will describe the invention in connection with preferred and alternative embodiments, it should be understand that the invention is not limited to those embodiments. Furthermore, one should understand that the drawings are not necessarily to scale. In certain instances, the applicant may have omitted details, which are not necessary for an understanding of the present invention.

### DETAILED DESCRIPTION OF THE INVENTION

The invention will next be described with reference to the figures wherein similar numbers indicate the same elements in all figures. Such figures are intended to be illustrative rather than limiting and are included herewith to facilitate the explanation of the apparatus of the present invention.

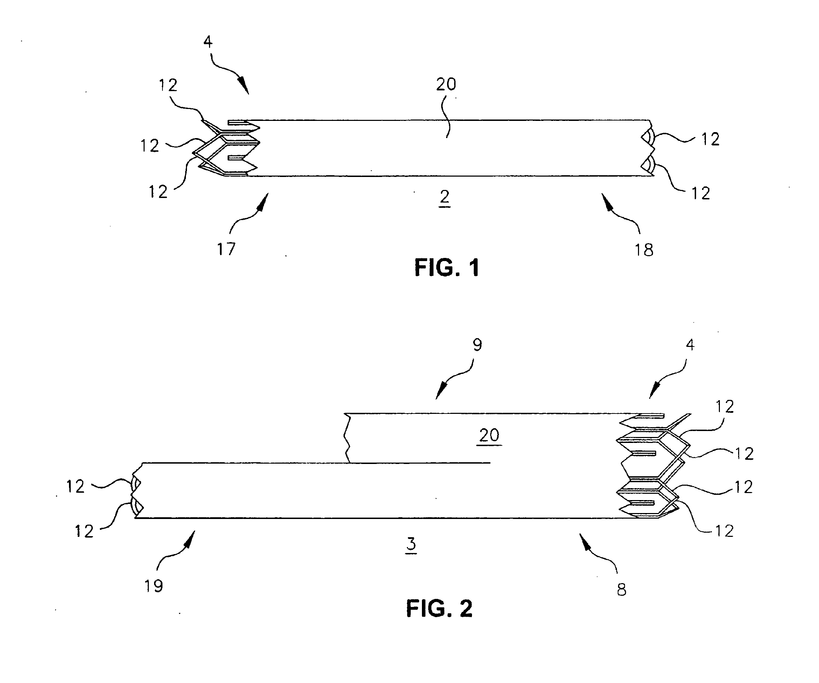

FIGS. 1 and 2 show a modular bifurcate stent graft comprising an iliac extension 2 and a bifurcate section 3, each having a stent covering 20, attached to intersecting stent members 12, which are helically braided to form a tubular stent section. The proximal end 18 of iliac extension 2 attaches to the socket 9 of bifurcate section 3 to form a complete bifurcate stent-graft. FIG. 1 shows an exemplary embodiment, where the iliac extension of a stent-graft comprises a covering 20 attached to intersecting stent members 12 at a distal end 17 of the iliac extension having alternating crossing intersections and crimped intersections. At proximal end 18 of the stent-graft, where covering 20 is attached, adjacent crossing intersections circumscribe the stent-graft. The bifurcated section 3, shown in FIG. 2, includes a trunk section 8 that bifurcates into a leg 19 adapted to extend into one iliac, and a socket 9 adapted to receive an iliac extension. Iliac extension 2 is a modular piece adapted to be inserted into the socket 9. The unbifurcated end of trunk 9, where covering 20 is attached, comprises alternating crossing intersections and crimped intersections. At the end of leg 19, covering 20 is attached to intersecting stent members at adjacent crossing intersections. At the end of socket 9, covering 20 is attached to adjacent crossing intersections having reversing portions of stent members or turns therebetween. The unbifurcated end of bifurcate section 3 and the distal end 17 of iliac extension 2 terminate with uncovered end segments 4 comprised of a circumferential series of hexagonal cells.

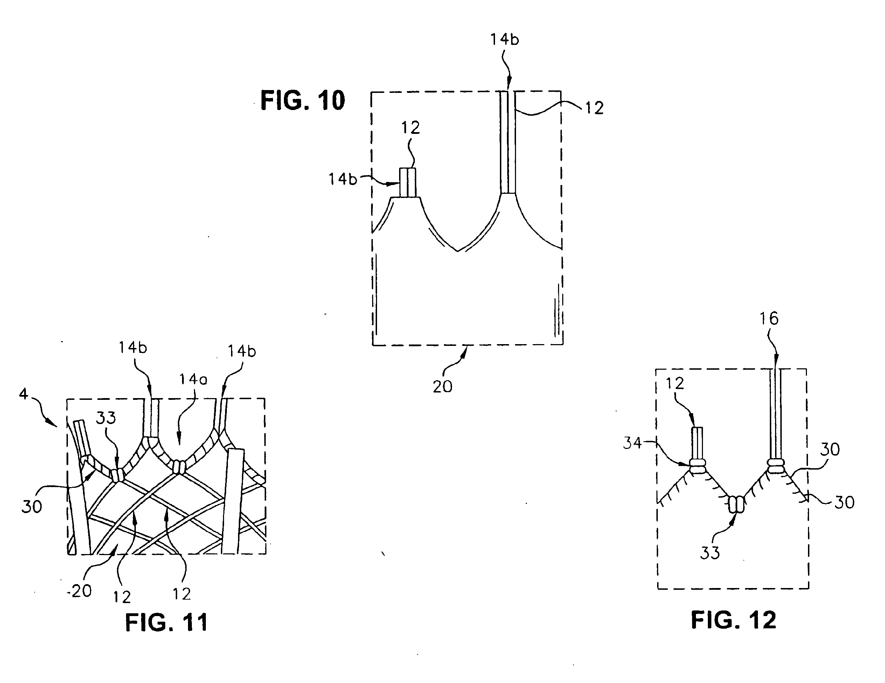

As best seen in FIG. 11 intersecting stent members 12 at the distal end 17 of iliac extension 2 (as seen looking radially outwardly) are braided to form a stent segment extending away from uncovered stent segment 4. The stent segment is formed by at least two continuous members 12 braided in a pattern, forming intersections 14. At each intersection 14, one member 12 is positioned radially outward relative to the other member 12. Following each member 12 along its helical path through the series of intersections 14, that member 12 may be in the radial inward position in one intersection 14 and in the radial outward position in the next intersection 14, or may be in the inward position for two intersections 14 and in the outward position for the next two, and so on. Exemplary braided stents 10 are disclosed, for example, in U.S. Pat. No. 4,655,771 to Hans I. Wallsten. The covering 20 attached to stent members 12 seen in Fig. 11 and the attachment between cover 20 and stent members 12 are described in more detail below.

Bifurcated section 3 (shown in Fig. 2) also comprises a braided construction of stent members 12. An exemplary braided stent section comprises a first set of stent members wound in a first helical direction and a second set of stent members wound in a second, opposite helical direction, forming a plurality of intersections. The first and second sets of stent members may be continuous stent members reversing axial direction at the ends of leg 19 and socket 9. Stent members 12 may be wire, such as nitinol or stainless steel, or may comprise polymer or any other stent material known in the art.

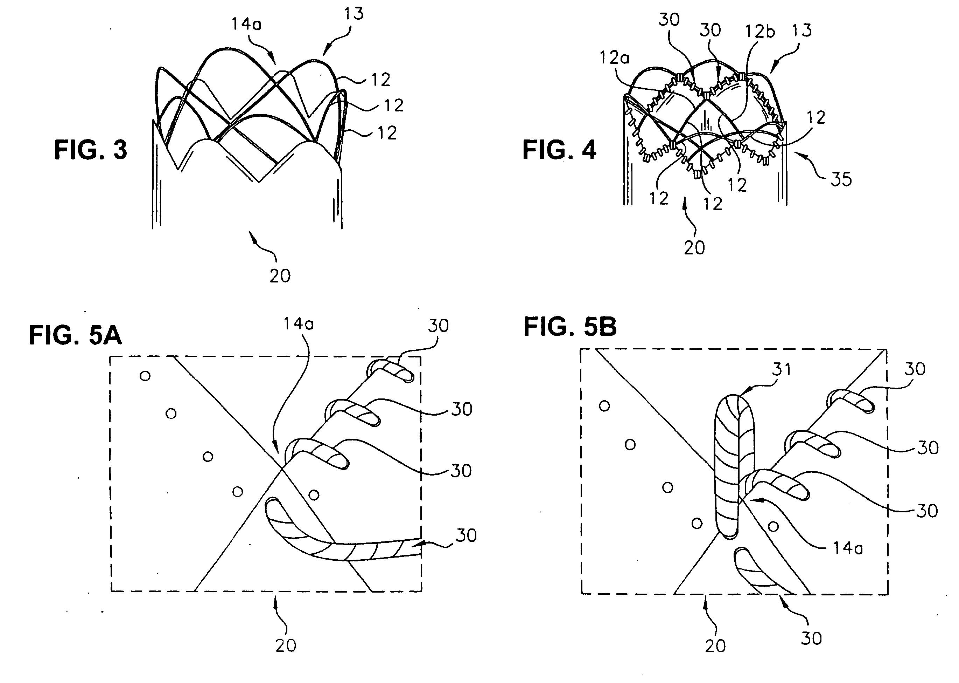

FIGS. 3 and 4 show an exemplary attachment, such as for the proximal end 18 of the iliac extension 2 (shown in FIG. 1), where the stent members 12 reverse axial direction to form a turn 13 and continue in the opposite direction. Stent members 12 pass over one another to form crossing intersections 14a. The covering 20 is trimmed to conform to the stent members 12, as shown in FIG. 3. When trimming at a first stent member 12a is completed, covering 20 may be stitched or lashed with a continuous filament 30 to the stent member. The filament 30 passes through the stent covering 20 and wraps around first stent member 12a, lashing covering 20 to first stent member 12a. This lashing is repeated until a crossing intersection 14a is reached. Then, continuous filament 30 is knotted around intersecting first 12a and second 12b stent members at a crossing intersection 14a. After continuous filament 30 is knotted at intersection 14a, it is lashed to second stent member 12b. FIG. 3 shows the covering 20 trimmed to follow the contour of the stent members 12. FIG. 4 shows a finished attachment with the continuous filament 30 knotted around the intersecting stent members 12 at crossing intersections 14a circumscribing a section of a stent-graft and lashed to stent members between adjacent crossing intersections. The filament 30 can be a suture or a wire, or other material having sufficient flexibility for lashing and knotting and sufficient strength to attach a covering on a stent.

FIGS. 5a-5e illustrate sequential steps of a method for attaching the covering 20 to a first stent configuration with a continuous lashing and knotting technique according to an exemplary embodiment of the present invention. In the first stent configuration, shown in Figs. 5a -5e, stent members 12 have turns 13 where they change axial direction. In the exemplary embodiment of the invention illustrated in FIGS. 5a-5e, covering 20 does not extend to turns 13, but is attached at and between adjacent crossing intersections circumscribing the end of the stent-graft section (in FIGS 5a-5e the proximal end of the iliac extension from FIG.1 is illustrated).

As seen in FIG. 5a, the continuous filament 30 lashes covering 20 to a first stent member 12a (as shown in FIG. 4). The lashing comprises passing filament 30 through covering 20, then around first stent member 12a, and back through covering 20. The lashing is repeated to form continuous stitches or lashings until intersection 14a is reached.

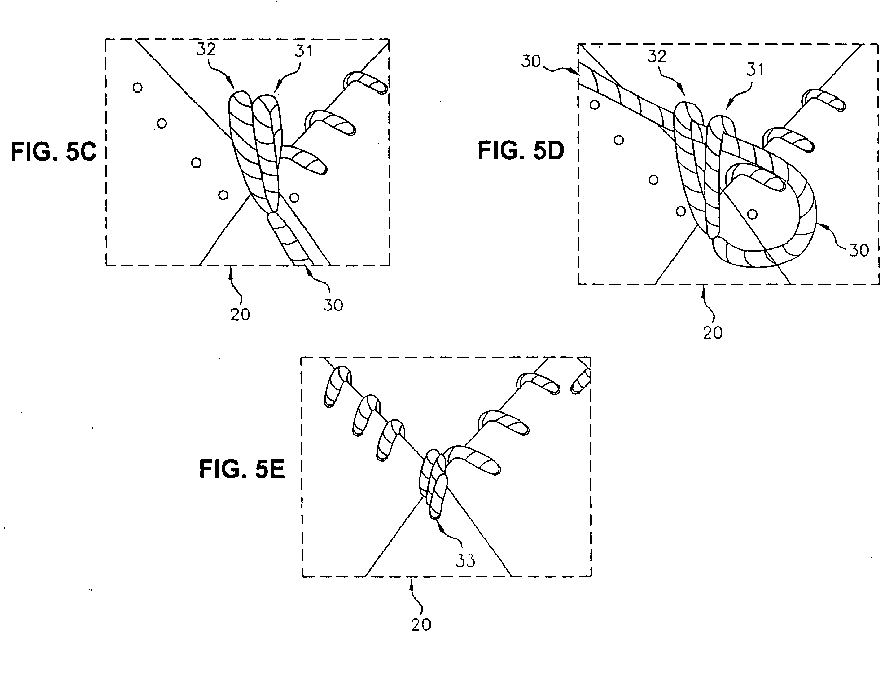

Next, as shown in FIG. 5b, a first loop 31 is made longitudinally around intersecting stent members 12 at intersection 14a with filament 30. First loop 31 encircles both intersecting stent members 12a and 12b at intersection 14a, allowing the intersecting members 12 to pivot relative to each other while fixing covering 20 at intersection 14a. In the exemplary embodiment illustrated in FIG. 5b, continuous filament 30 is passed through covering 20 from the inside of stent 10 to the outside of stent 10 at approximately a first apex formed by intersecting stent members 12 and looped around intersection 14a at approximately a longitudinally opposite apex formed by intersecting stent members 12. Then, continuous filament 30 is again passed through covering 20 proximate the first apex. FIG. 5c shows a second loop 32 made around the intersection 14a with filament 30. Second loop 32 is formed in the same way as first loop 31.

FIG. 5d shows filament 30 passing through the first 31 and second 32 loop. Then, filament 30 is pulled tight to form a longitudinal knot 33 to fix covering 20 to intersecting stent members 12 at intersection 14a (shown in Fig. 5e).

Finally, as shown in FIG. 5e continuous filament 30 is lashed to second stent member 12b (shown in Fig. 4).

Referring again to Fig. 5a, continuous filament 30 is passed through the covering 20. Prior to beginning the lashing, a knot is made to secure the filament 30 to the covering 20 and stent 10.

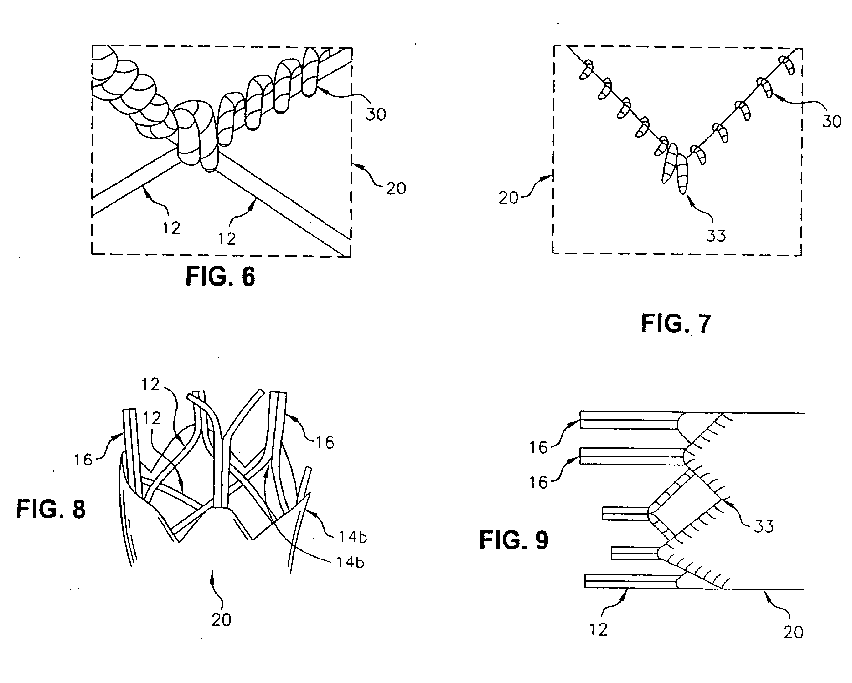

FIGS. 6 and 7 show an inside view and outside view, respectively, of the attachment between intersecting stent members 12 and the stent covering 20 at a crossing intersection 14a. Covering 20 is attached to intersecting stent members 12 of a stent-graft by consecutive lashings of continuous filament 30 and a longitudinal knot 33. The attachment is positioned at an end of a braided stent section formed by the stent members 12.

FIGS. 8 and 9 show an exemplary attachment for a stent configuration comprising alternate crossing intersections 14a and crimped intersections 14b, such as for the distal end 17 of iliac extension 2 (shown in FIG. 1) or the unbifurcated end of bifurcate section 3 (shown in FIG. 2). At crimped intersection 14b, stent members 12 meet and are secured together with a crimp 16. FIG. 8 shows covering 20 trimmed to conform to stent members 12 at an end of a stent section comprising alternate crimped intersections and crossing intersections. FIG. 9 shows the completed attachment of covering 20 to intersecting stent members 12 at an end of a stent section comprising alternate crimped intersections and crossing intersections. The attachment of the present embodiment comprises lashings of a continuous filament 30 onto stent members 12, longitudinal knot 33 formed in continuous filament 30 at crossing intersections 14a, and lateral knot 34 formed in continuous filament 30 directly below crimp 16 at crimped intersections 14b of stent members 12.

FIGS. 10-12 show a method for forming an attachment of covering 20 to intersecting stent members 12, where intersecting stent members alternately form crossing intersections 14a and crimped intersections 14b at the end of a braided section of a stent. FIG. 10 shows the proximal end 18 of the bifurcate section 3 (shown in Fig. 2) with crimped intersections 14b. As shown in FIG. 10, covering 20 is contoured or trimmed to conform to stent members 12 at the end of a stent section comprising alternate crimped intersections and crossing intersections.

FIGS. 11 and 12 shows the completed attachment from inside and outside the stent, respectively. Continuous filament 30 is knotted at a plurality of adjacent intersections 14 circumscribing the stent section formed by stent members 12. As shown in FIG. 11, continuous filament 30 repeatedly passes through the covering 20 and loops around portions of stent members 12 interconnecting the adjacent intersections 14. Then, when continuous filament 30 reaches a crossing intersection 14a, a longitudinal knot 33 (shown in Fig. 12) is formed as described above. After knot 33 is formed in continuous filament 30, continuous filament 30 is again repeatedly passed through covering 20 and looped around another portion of a stent member 12 interconnecting the adjacent intersections 14. Then, when continuous filament 30 reaches a crimped intersection 14b, a lateral knot 34 (shown in Fig. 12) is formed. Lateral knot 34 is formed by forming two loops of continuous filament 30 around both stent members at crimped intersection 14b, capturing covering 20 in the loops. Continuous filament 30 is then passed through the loops and drawn tight to form knot 34.

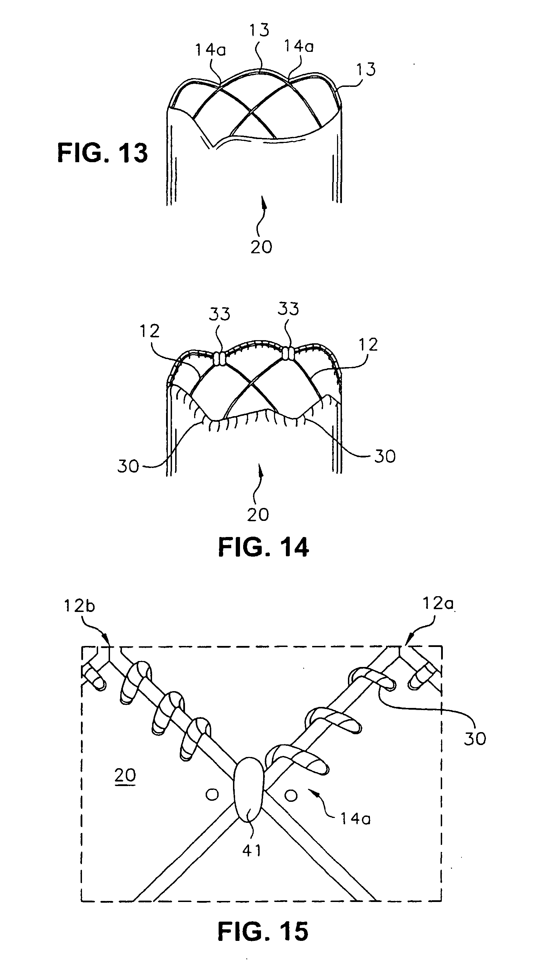

FIGS. 13 and 14 show an exemplary attachment, such as for the end of socket 9 of bifurcate section 3 (shown in Fig. 2), where the stent members 12 form turns 13 and covering 20 is attached to the scalloped end of a stent graft section formed by turns 13. As shown in FIG. 13, covering 20 is contoured or trimmed to match the stent members 12 at turns 13. As shown in FIG. 13, the contoured ends of covering 20 coincide with the scalloped ends formed by turns 13 in stent members 12.

Continuous filament 30 is knotted at a plurality of adjacent crossing intersections 14a circumscribing the stent formed by stent members 12. As shown in FIG. 14, continuous filament 30 repeatedly passes through the covering 20 and loops around turns 13 in stent members 12. Then, when continuous filament 30 reaches a crossing intersection 14a, a longitudinal knot 33 is formed as described above. After knot 33 is formed in continuous filament 30, continuous filament 30 is again repeatedly passed through covering 20 and looped around another turn 13 formed in stent members 12.

An exemplary stent, is configured to treat an abdominal aortic aneurysm (AAA), comprising a bifurcate section 3 with an iliac extension 2 connected to the socket 9 of bifurcate section 3. Iliac extension 2 and bifurcate section 3 are illustrated in FIGS. 1 and 2, respectively. The exemplary stent comprises various embodiments of the attachment according to the present invention. An attachment comprising lashings, longitudinal knots 33 and lateral knots 34 (as shown in Fig. 12) is provided at the unbifurcated end of trunk 8 of bifurcate section 3 and at the proximal end 17 of iliac extension 2. An attachment comprising lashings and longitudinal knots 33 (as shown in Fig. 4) is provided at proximal end 18 of iliac extension 2 (shown in Fig. 1) and the distal end of leg 19 of bifurcate section 3 (shown in Fig. 2). An attachment comprising lashings extending around scalloped turns 13 and longitudinal knots 33 (as shown in Fig. 14) is provided at the distal end of socket 9 of bifurcate section 3 (shown in Fig. 2).

Referring now to FIG. 15, covering 20 may also be attached to stent members 12 using a combination of a continuous filament 30 and a pivotal restraint member 41 according to an alternative exemplary embodiment of the invention. The covering 20 is trimmed to conform to the stent members 12, as shown in FIG. 3. When trimming at a first stent member 12a is completed, covering 20 may be stitched or lashed with a continuous filament 30 to the stent member. The filament 30 passes through the stent covering 20 and wraps around first stent member 12a, lashing covering 20 to first stent member 12a. This lashing is repeated until a crossing intersection 14a is reached. Then, continuous filament 30 is lashed to second stent member 12b. The continuous filament 30 lashes cover 20 to the intersecting stent members 12 between crossing intersections 14a to circumscribe a section of a stent-graft, preferably at its end. The filament 30 can be a suture or a wire, or other material having sufficient flexibility for lashing and sufficient strength to lash a covering on a stent. At crossing intersections 14a, adjacent the lashing, pivotal restraint member 41 is disposed around both stent members forming the intersection and through cover 20. The pivotal restraint member may be a knot formed in a filament separate from continuous filament 30. Alternatively, the pivotal restraint may be a ring, staple, or other structure that fixes the filaments at their intersection while allowing them to pivot relative to each other.

Although the invention is illustrated and described herein with reference to specific embodiments, the invention is not intended to be limited to the details shown. Rather, various modifications may be made in the details within the scope and range of equivalents of the claims and without departing from the invention.

## Claims

1. A stent-graft comprising: a tubular stent formed by intersecting stent members (12), a stent covering (20) covering the tubular stent, and an attachment comprising a filament (30) having been repeatedly passed through said stent covering (20) and wrapped around at least one of said stent members (12); a restraint member encircled about at least one intersection (14) of said intersecting stent members (12) and attached to the covering (20) at said intersection (14), **characterized by** the intersecting stent members (12) being helically braided at at least one intersection (14) and the restraint member being positioned at at least one end of a braided section of the stent formed by said stent members (12).

2. The stent-graft of claim 1 herein said restraint member comprises a portion of said filament (30) knotted around said intersecting stent members (12) at one or more intersections (14) of said intersecting stent members (12).

3. The stent-graft of claim 2 wherein said filament (30) is a suture.

4. The stent-graft of claim 2 wherein said filament (30) is a wire.

5. The stent-graft of claim 2 wherein said filament (30) is knotted at a plurality of adjacent intersections (14) circumscribing a stent formed by said stent members (12).

6. The stent-graft of claim 5 wherein said filament (30) is looped around portions of the stent members (12) interconnecting the adjacent intersection (14).

7. The stent-graft of claim 6 wherein the ends of said covering (20) follow the contour of said stent members (12) between adjacent interconnected intersections (14).

8. The stent-graft of claim 2 wherein the covering (20) comprises laminated layers of a woven dacron and a thin expanded polytetrafloroethlene.

9. The stent-graft of claim 2 wherein said filament (30) forms a plurality of sequential loops around each of a cicumferential series of said intersecting stent members (12).

10. The stent-graft of claim 1 wherein said restraint member is a knot formed in a filament separate from said filament (30).

11. The stent-graft of claim 1 wherein said restraint member is a ring.

12. The stent-graft of claim 1 wherein said restraint member is a staple.

13. The stent-graft of claim 1 wherein said restraint member encircles said intersecting stent members (12) longitudinally.

14. A method of attaching a covering (20) to a stent, said method comprising the steps of:
providing a stent frame formed by intersecting stent members (12);
positioning the covering (20) to the frame;
repeatedly passing a filament (30) through the stent covering (20) along one of said stent members (12) and wrapping the filament (30) around said stent member (12) between said repeated passings;
**characterized by** encircling the filament (30) around an intersection (14) of said stent member (12) with a second stent member (12), thereby forming a restraint member;
positioning the restraint member at at least one end of a braided section of the stent formed by the stent members (12), wherein the intersecting stent members (12) are helically braided at at least one intersection (14).

15. The method of claim 14 wherein the filament (30) is a suture.

16. The method of claim 14 wherein the filament (30) is a wire.

17. The method of claim 14, further comprising the step of wrapping said filament (30) around a succession of stent members (12) at an end of the stent formed by the stent members (12) and knotting said filament (30) at a plurality of adjacent intersections (14) circumscribing the stent end.

18. The method of claim 16 wherein the stent member wrapping and the end of the covering (20) follow the contour of said successive stent members (12).

19. The method of claim 14 wherein the covering (20) comprises laminated layers of a woven dacron and a thin expanded polytetrafloroethlene.

20. The method of claim 14 wherein each of the wrappings comprises one of several sequential wrappings on the respective stent member (12).

## Patentansprüche

1. Stentgraft umfassend: einen röhrenförmigen Stent, gebildet durch sich überkreuzende Stentelemente (12), eine den röhrenförmigen Stent abdeckende Stentabdeckung (20), und eine Befestigung, umfassend ein Filament (30), welches mehrmals durch die Stentabdeckung (20) hindurch geführt wurde und um mindestens eines der Stentelemente (12) herum gewickelt wurde; ein Halteelement, das über mindestens einen Schnittpunkt (14) der sich überkreuzenden Stentelemente (12) herum geschlungen ist und an dem Schnittpunkt (14) an der Abdeckung (20) befestigt ist, **dadurch gekennzeichnet, dass** die sich überkreuzenden Stentelemente (12) an mindestens einem Schnittpunkt (14) wendelförmig geflochten sind, und das Halteelement an mindestens einem Ende eines geflochtenen Abschnitts des durch die Stentelemente (12) gebildeten Stents positioniert ist.

2. Stentgraft gemäß Anspruch 1, wobei das Halteelement einen Abschnitt des Filaments (30) umfasst, der an einem oder mehreren Schnittpunkten (14) der sich überkreuzenden Stentelemente (12) um die sich überkreuzenden Stentelemente (12) herum geknotet ist.

3. Stentgraft gemäß Anspruch 2, wobei das Filament (30) eine Wundnaht ist.

4. Stentgraft gemäß Anspruch 2, wobei das Filament (30) ein Draht ist.

5. Stentgraft gemäß Anspruch 2, wobei das Filament (30) an einer Vielzahl von aneinander angrenzenden Schnittpunkten (14) geknotet ist, welche einen durch die Stentelemente (12) gebildeten Stent abgrenzen.

6. Stentgraft gemäß Anspruch 5, wobei das Filament (30) um Teile der Stentelemente (12) herum gewunden ist, welche die aneinander angrenzenden Schnittpunkte (14) miteinander verbinden.

7. Stentgraft gemäß Anspruch 6, wobei die Enden der Abdeckung (20) dem Umriss der Stentelemente (12) zwischen aneinander angrenzenden, miteinander verbundenen Schnittpunkten (14) folgen.

8. Stentgraft gemäß Anspruch 2, wobei die Abdeckung (20) laminierte Schichten eines gewobenen Dacrons und eines dünnen expandierten Polytetrafluorethylens umfasst.

9. Stentgraft gemäß Anspruch 2, wobei das Filament (30) eine Vielzahl von aufeinander folgenden Windungen um jedes der sich überkreuzenden Stentelemente (12) aus einer Reihe in Umfangsrichtung bildet.

10. Stentgraft gemäß Anspruch 1, wobei das Halteelement ein Knoten ist, der in einem Filament getrennt von dem Filament (30) ausgebildet ist.

11. Stentgraft gemäß Anspruch 1, wobei das Halteelement ein Ring ist.

12. Stentgraft gemäß Anspruch 1, wobei das Halteelement eine Klammer ist.

13. Stentgraft gemäß Anspruch 1, wobei das Halteelement die sich überkreuzenden Stentelemente (12) in Längsrichtung umschlingt.

14. Verfahren zum Befestigen einer Abdeckung (20) an einem Stent, wobei das Verfahren umfasst die Schritte von:
Bereitstellen eines durch sich überkreuzende Stentelemente (12) gebildeten Stentrahmens;
Positionieren der Abdeckung (20) in Bezug auf den Rahmen;
mehrmaliges hindurch führen eines Filamentes (30) durch die Stentabdeckung (20) entlang einem der Stentelemente (12) und Wickeln des Filamentes (30) um das Stentelement (12) herum zwischen den wiederholten Durchgängen;
**gekennzeichnet durch** Schlingen des Filamentes (30) um einen Schnittpunkt (14) des Stentelementes (12) mit einem zweiten Stentelement (12) herum, wodurch ein Halteelement gebildet wird; Positionieren des Halteelementes an mindestens einem Ende eines geflochtenen Abschnitts des **durch** die Stentelemente (12) gebildeten Stents, wobei die sich überkreuzenden Stentelemente (12) an mindestens einem Schnittpunkt (14) wendelförmig geflochten sind.

15. Verfahren gemäß Anspruch 14, wobei das Filament (30) eine Wundnaht ist.

16. Verfahren gemäß Anspruch 14, wobei das Filament (30) ein Draht ist.

17. Verfahren gemäß Anspruch 14, ferner umfassend den Schritt, das Filament (30) um eine Abfolge von Stentelementen (12) an einem Ende des durch die Stentelemente (12) gebildeten Stents herum zu wickeln und das Filament (30) an einer Vielzahl von aneinander angrenzenden, das Stentende begrenzenden Schnittpunkten (14) zu verknoten.

18. Verfahren gemäß Anspruch 14, wobei die Stentelement-Umwicklung und das Ende der Abdeckung (20) dem Umriss der aufeinander folgenden Stentelemente (12) folgen.

19. Verfahren gemäß Anspruch 14, wobei die Abdeckung (20) laminierte Schichten eines gewobenen Dacrons und eines dünnen expandierten Polytetrafluorethylens umfasst.

20. Verfahren gemäß Anspruch 14, wobei jede der Umwicklungen eine von mehreren aufeinander folgenden Umwicklungen auf dem jeweiligen Stentelement (12) umfasst.

## Revendications

1. Stent-greffon comprenant: un Stent tubulaire, formé par des éléments de Stent (12) s'entrecroisants, un recouvrement de Stent (20) recouvrant le Stent tubulaire, et un fixage, comprenant un filament (30), qui a été fait passer à travers le recouvrement de Stent (20) à maintes fois et qui était enroulé autour d'au moins un des éléments de Stent (12); un élément d'encastrement encirclé au-dessus d'au moins une intersection (14) des éléments de Stent (12) s'entrecroisants et attaché au recouvrement (20) dans l'intersection (14), **caractérisé en ce que** les éléments de Stent (12) s'entrecroisants sont tressés de façon hélicoïdale dans au moins une intersection (14) et que l'élément d'encastrement est positionné dans au moins une extrémité d'une partie tressée du Stent formé par les éléments de Stent (12).

2. Stent-greffon selon la revendication 1, dans lequel l'élément d'encastrement comprend une partie du filament (30) qui est nouée autour des éléments de Stent (12) s'entrecroisants dans une ou plusieurs intersections (14) des éléments de Stent (12) s'entrecroisants .

3. Stent-greffon selon la revendication 2, dans lequel le filament (30) est une soudure de blessure.

4. Stent-greffon selon la revendication 2, dans lequel le filament (30) est un fil.

5. Stent-greffon selon la revendication 2, dans lequel le filament (30) est noué dans une pluralité d'intersections (14) adjacentes qui circonscrivent un Stent formé par les éléments de Stent (12).

6. Stent-greffon selon la revendication 5, dans lequel le filament (30) est enroulé autour des parties des éléments de Stent (12) qui interconnectent l'intersection (14) adjacente.

7. Stent-greffon selon la revendication 6, dans lequel les extrémités du recouvrement (20) suivent le contour des éléments de Stent (12) entre des intersections (14) adjacentes interconnectées.

8. Stent-greffon selon la revendication 2, dans lequel le recouvrement (20) comprend des couches laminées d'un dacron tissé et d'un polytetrafluoréthylène étiré effilé.

9. Stent-greffon selon la revendication 2, dans lequel le filament (30) forme une pluralité de boucles séquentiels autour de chacun d'une série circonférentielle des éléments de Stent (12) s'entrecroisants.

10. Stent-greffon selon la revendication 1, dans lequel l'élément d'encastrement est un noeud qui est formé dans un filament séparé du filament (30).

11. Stent-greffon selon la revendication 1, dans lequel le filament d'encastrement est un anneau.

12. Stent-greffon selon la revendication 1, dans lequel le filament d'encastrement est une agrafe.

13. Stent-greffon selon la revendication 1, dans lequel le filament d'encastrement encircle les éléments de Stent (12) s'entrecroisants de façon longitudinale.

14. Procédé de fixation d'un recouvrement (20) sur un Stent, le procédé comprenant les étapes de:
fournir un cadre de Stent formé par des éléments de Stent (12) s'entrecroisants;
positionner le recouvrement (20) par rapport au cadre;
faire passer à maintes fois un filament (30) à travers le recouvrement de Stent (20) le long d'un des éléments de Stent (12), et enrouler le filament (30) autour du filament de Stent (12) entre les passes répétées;
**caractérisé en ce que** le filament (30) est encirclé autour d'une intersection (14) de l'élément de Stent (12) avec un deuxième élément de Stent (12), par quoi est formé un élément d'encastrement ; et que l'élément d'encastrement est positionné dans au moins une extrémité de la partie tressée du Stent formé par les éléments de Stent (12), les éléments de Stent (12) s'entrecroisants étant tressés de façon hélicoïdale dans au moins une intersection (14).

15. Procédé selon la revendication 14, dans lequel le filament (30) est une soudure de blessure.

16. Procédé selon la revendication 14, dans lequel le filament (30) est un fil.

17. Procédé selon la revendication 14, comprenant en outre l'étape d'enrouler le filament (30) autour d'une succession d'éléments de Stent (12) dans une extrémité du Stent formé par les éléments de Stent (12) et de nouer le filament (30) dans une pluralité d'intersections (14) adjacentes qui circonscrivent l'extrémité du Stent.

18. Procédé selon la revendication 14, dans lequel l'enroulement d'éléments de Stent et l'extrémité du recouvrement (20) suivent le contour des éléments de Stent (12) successifs.

19. Procédé selon la revendication 14, dans lequel le recouvrement (20) comprend des couches laminées d'un dacron tissé et d'un polytetrafluoréthylène étiré effilé.

20. Procédé selon la revendication 14, dans lequel chacun des enroulements comprend un de plusieurs enroulements séquentiels sur l'élément de Stent (12) respectif.
